# EUROPEAN PATENT SPECIFICATION

(11) **EP 1 443 915 B1**
(45) Date of publication and mention of the grant of the patent: **21.06.2006**
(21) Application number: 02761067.4
(22) Date of filing: 16.08.2002
(51) Int. Cl.: A61K 31/11, A61K 31/192, A61K 31/277, A61K 31/12, A61K 31/075, A61Q 19/00, A61Q 17/00, A61P 17/00, A61P 17/02, A61P 17/04, A61K 8/33

(54) **PHARMACEUTICAL AND COSMETIC COMPOSITIONS CONTAINING OXY GROUP-BEARING AROMATIC ALDEHYDES**
PHARMAZEUTISCHE UND KOSMETISCHE ZUSAMMENSETZUNGEN MIT OXY-GRUPPEN-TRAGENDEN AROMATISCHEN ALDEHYDEN
COMPOSITIONS PHARMACEUTIQUES ET COSMETIQUES CONTENANT DES ALDEHYDES AROMATIQUES PORTEURS DE GROUPES OXY

(30) Priority: 16.11.2001 US 332277 P; 04.01.2002 US 346049 P; 01.04.2002 US 368518 P; 01.04.2002 US 368565 P; 30.05.2002 US 384589 P; 30.05.2002 US 384690 P
(43) Date of publication of application: 11.08.2004
(73) Proprietor: Cutanix Corporation, Los Altos, CA 94022 (US)
(72) Inventor: ENGLES, Charles, R., Portola Valley, CA 94028 (US); FULLER, Bryan, Edmond, OK 73104 (US); PILCHER, Brian, Keith, Edmond, OK 73034 (US)
(74) Representative: Wibbelmann, Jobst
(86) International application number: PCT/US2002/021844
(87) International publication number: WO 2003/043621

(56) References cited:
- EP-A- 0 193 257
- EP-A- 0 395 441
- EP-A- 0 635 208
- EP-A- 0 985 408
- WO-A-92/09276
- WO-A-97/47280
- GB-A- 1 545 954
- GB-A- 2 244 645
- US-A- 414 770
- US-A- 4 202 877
- US-A- 4 714 609
- US-A- 5 614 179
- US-A- 5 626 852
- US-A- 5 668 182
- US-A- 5 733 535
- US-A- 6 086 903
- US-A- 6 126 930
- US-B1- 6 204 229
- US-B1- 6 214 879
- FELTON ET AL.: "New class of broad spectrum antibacterials" TGA COSMETIC J., vol. 2, no. 1, 1970, pages 16-19, XP008010025
- FARAH ET AL.: "Pharmacologically active prenylpropanoids from Senra incana" PLANTA MEDICA, vol. 58, no. 1, 1992, pages 14-18, XP008010128
- VAN DEN WORM ET AL.: "Effects of methoxylation of apocyanin and analogs on the inhibition of reactive oxygen species production by stimulated human neutrophils" EUR. J. PHARMACOL., vol. 433, no. 2-3, 2001, pages 225-230, XP001126258
- CHANG ET AL.: "Benzyloxybenzaldehyde analogues as novel adenyl cyclase activators" BIOORG. MED. CHEM. LETTERS, vol. 11, no. 15, 2001, pages 1971-1974, XP002221475
- DATABASE WPI Section Ch, Week 199412 Derwent Publications Ltd., London, GB; Class B04, AN 1994-097768 XP002221477 & JP 06 048929 A (SHISEIDO CO LTD), 22 February 1994 (1994-02-22)
- DATABASE WPI Section Ch, Week 200145 Derwent Publications Ltd., London, GB; Class B05, AN 2001-420810 XP002221478 & JP 2001 106639 A (TAISHO PHARM CO LTD), 17 April 2001 (2001-04-17)
- DATABASE WPI Section Ch, Week 199546 Derwent Publications Ltd., London, GB; Class A96, AN 1995-355186 XP002221479 & JP 07 242558 A (TANAKA A), 19 September 1995 (1995-09-19)
- DATABASE WPI Section Ch, Week 199603 Derwent Publications Ltd., London, GB; Class B05, AN 1996-026967 XP002221480 & JP 07 300412 A (POLA CHEM IND INC), 14 November 1995 (1995-11-14)
- PATENT ABSTRACTS OF JAPAN vol. 004, no. 081 (C-014), 11 June 1980 (1980-06-11) & JP 55 045656 A (RIKAGAKU KENKYUSHO;OTHERS: 02), 31 March 1980 (1980-03-31)
- PATENT ABSTRACTS OF JAPAN vol. 004, no. 081 (C-014), 11 June 1980 (1980-06-11) & JP 55 045659 A (RIKAGAKU KENKYUSHO;OTHERS: 02), 31 March 1980 (1980-03-31) -& DATABASE CAPLUS [Online] CHEMICAL ABSTRACTS SERVICE, COLUMBUS, OHIO, US; Database accession no. 1980:610255 XP002222244
- PATENT ABSTRACTS OF JAPAN vol. 1995, no. 04, 31 May 1995 (1995-05-31) & JP 07 002640 A (MARINO FORUM 21), 6 January 1995 (1995-01-06)
- PATENT ABSTRACTS OF JAPAN vol. 005, no. 062 (C-052), 25 April 1981 (1981-04-25) & JP 56 012310 A (RIKAGAKU KENKYUSHO;OTHERS: 02), 6 February 1981 (1981-02-06)
- DATABASE WPI Derwent Publications Ltd., London, GB; AN 1977-85942y XP002043902 & JP 52 044375 B (ICHIMARU BOEKI KK) 28 November 1977 (1977-11-28)
- DATABASE CAPLUS [Online] CHEMICAL ABSTRACTS SERVICE, COLUMBUS, OHIO, US; retrieved from STN, accession no. 1995:370840 Database accession no. 122:196554 XP002221476 & JP 06 329516 A (MIKIMOTO SEIYAKU KK) 29 November 1994 (1994-11-29)

## Description

### BACKGROUND OF THE INVENTION

### Field of the Invention

This invention relates to oxy group-bearing aromatic aldehydes and their use as active ingredients in cosmetics and pharmaceuticals. More particularly it concerns such aldehydes and their use in cosmetics and as topical, transdermal or systemic pharmaceuticals.

### State of the Art

This invention involves the use of aromatic aldehydes. Many aromatic aldehydes are known materials that commonly find use as chemical intermediates. Some aromatic aldehydes are components of natural products as well.

The present invention uses these aldehydes as active ingredients in pharmaceuticals and cosmetics. While the invention contemplates that these aldehyde materials can find application as systemic agents against inflammatory conditions when delivered transdermally or orally or by injection, at this time their preferred uses are as components of topical cosmetic and pharmaceutical compositions used to treat a wide range of dermatological conditions ranging from dermatitis and U.V. - induced inflammation through psoriasis and acne.
The aromatic aldehides used in the present invention are selected from the group consisting of 2-ethoxybenzaldehyde, 4-ethoxybenzaldehyde, 4-propoxybenzaldehyde, 4-butoxybenzaldehyde, 4-pentyloxybenzaldehyde, and 4-hexyloxybenzaldehyde.

Therapies used in the past to deal with conditions such as eczema and psoriasis have included the use of simple emollients. Topical steroids ranging from mild agents such as hydrocortisone (1%) through more potent materials such as clobetasol propionate (0.05%) have been indicated with the common inflammatory dermatoses. In addition, corticosteroids and immunosuppressants have been used to treat skin conditions. Vitamin D and its derivatives such as calcipotrial and tacolcitol and vitamin A and other retinoids have been used to treat dermatological problems. The vitamin D materials are used to treat acne.

In addition to those directly topical therapies, it is well known that many materials pass through the skin and enter the systemic circulation when placed on the skin. The line between "topical" and this so called "transdermal" administration of drugs is a fuzzy one and many therapies heretofore have had both topical and transdermal aspects.

These therapies are not without their limitations. Emollients are very temporary and must be repeatedly renewed. Topical steroid use is associated with thinning skin, bruising, and rashes as well as serious systemic side effects such as development of Cushing's Syndrome.

The vitamin D materials often pass transdermally and can have unexpected effects on the user's systemic calcium metabolism. The retinoids are reported to cause acne in some cases and to produce teratogenic effects if absorbed transdermally during pregnancy.

It is clear that there is a need for additional topical compositions which can effectively treat dermatological conditions. It would be highly desirable if these compositions could also treat optionally transdermally or otherwise systemically treatable inflammatory conditions and avoid some or all of the problems associated with therapies now in use.
Patent application EP 0 985 408 relates to combinations particularly for cosmetical and dermatological purposes containing an adhesive agent and an antimicrobial agent. Anisic aldehyde (4-methoxybenzaldehyde) is disclosed as one possible microbiozide. These compositions can be used for treatment of phenomena like arm pit odour, body odour, food odour, head odour, akne, seborrhoic dermatitis, microbial superinfections in relation with exzema, psoriasis and immuno suppression.
US 5,733,535 relates to topical compositions comprising N-acetylcysteine or related compounds, which may contain perfume chemicals for use in odour masking as e.g. anisic aldehyde (4-methoxybenzaldehyde). This compound is also suggested as a perfumery agent in US 6,126,930 relating to personal care and pharmaceutical compositions comprising an alcohol, a personal care polymer, and an alcohol-masking perfumery component. EP 0 635 208 relates to louse repellent compositions containing a methoxy-substituated aromatic compound, e.g. anisic aldehyde.
WO 92/09276 discloses aromatic aldehydes, their derivatives and pharmaceutical compositions thereof for the treatment of cells having an abnormally elevated cell proliferation which results in diseases such as psoriasis, inflammatory diseases, rheumatic diseases and allergic dermatologic reactions. However, a particular effect of alkoxy-substituated benzaldehydes is not disclosed therein.
In addition, a carcinostatic effect of substituated benzaldehydes is imputed by JP-A-55045656 and JP-A-56012310, among which also specific alkoxy benzaldehydes are disclosed.

### SUMMARY OF THE INVENTION

It has now been found that a group of oxy group-bearing aromatic aldehydes are effective topical agents against inflammation-related dermatological conditions. These aldehydes also appear to be delivered to a measurable extent transdermally and thus to potentially achieve systemic and/or localized anti-inflammatory effects within the body. In view of these findings, it further appears that these aldehydes can be effective against other inflammatory conditions when administered by other systemic routes.

In one of its composition aspects, this invention is directed to topical pharmaceutical and cosmetic compositions containing a pharmaceutically or cosmetically acceptable topical carrier and a pharmaceutically or cosmetically effective amount of one or more aromatic aldehyde compounds selected from the group of 2-ethoxybenzaldehyde, 4-ethoxybenzaldehyde, 4-propoxybenzaldehyde, 4-butoxybenzaldehyde, 4-pentyloxybenzaldehyde, and 4-hexyloxybenzaldehyde.

In another of its composition aspects, this invention is directed to pharmaceutical compositions for topical, transdermal or other systemic administration containing a pharmaceutically-acceptable carrier and one or more of the aromatic aldehyde compounds selected from the group consisting of 2-ethoxybenzaldehyde, 4-ethoxybenzaldehyde, 4-propoxybenzaldehyde, 4-butoxybenzaldehyde, 4-pentyloxybenzaldehyde, and 4-hexyloxybenzaldehyde.

### DETAILED DESCRIPTION OF THE INVENTION

### Brief Description of the Drawing

**Figure 1:** A schematic diagram illustrating inflammatory processes in the skin and showing the relationship of inflammation to the release of various proteins.

**Figure 2:** A repeat of Figure 1 illustrating those inflammatory processes which are effectively treated using the present invention.

**Figure 3A and 3B and Figures 4A, 4B and 4C**: Bar graphs which show the effects of aldehydes employed in the compositions of this invention on interleukin 1"1L-1"-induced prostaglandin E2 "PGE₂" expression in dermal fibroblasts.

**Figure 5:** A bar graph which shows the effects of aldehydes employed in the compositions of this invention on tetradecanoyl phorbol acetate "TPA"-induced PGE₂ expression in keratinocytes.

**Figure 6:** A table which shows the effects of aldehydes employed in the compositions of this invention and other related compounds on expression levels of varius proteins in fibroblasts challenged with IL-1 or UV light (only compounds CX-5, 21, 22, 23, 24 and 25 are claimed).

**Figure 7:** A table which shows the effects of aldehydes employed in the compositions of this invention and other related compounds on expression levels of varius proteins in keratinocytes challenged with TPA or UV light (only compounds CX-5, 21, 22, 23, 24 and 25 are claimed).

**Figures 8A, 8B, 9A, 9B, 10A, 10B, 11A and 11B:** Bar graphs of data tabulated in Fig. 6 (only compounds CX-5, 21, 22, 23, 24 and 25 are claimed).

**Figures 12A, 12B, 13A, 13B, 14A and 14B:** Bar graphs of data tabulated in Fig. 7 (only compounds CX-5, 21, 24 and 25 are claimed).

**Figures 15A and 15B:** Bar graphs of data obtained in an in vivo test of the lotion formulation of Example 9.

### Definitions

"Ionizing radiation" refers to any radiation that ionizes the atoms or molecules of matter. It may consist of particles (such as electrons) or it may be electromagnetic (ultraviolet radiation; X-rays; gamma radiation). Ionizing radiation occurs naturally, for example as a component of sunlight, and is emitted by radioactive substances. It is also produced artificially in X-ray machines, particle accelerators, nuclear reactors, etc.

"Isolated", when used to define the state of purity of the aromatic aldehyde compounds used in the practice of this invention, means that the aromatic aldehyde has been substantially freed of (i.e at least about 90% and especially at least about 95% freed of) or separated from related feedstocks, co-products, or in the case of naturally-occurring mixtures, related materials with which the aldehyde appears in nature.

"Pharmaceutically-acceptable topical carrier" and equivalent terms refer to an inactive liquid or cream vehicle capable of suspending or dissolving the aromatic aldehyde and having the properties of being nontoxic and noninflammatory when applied to the skin. This term is specifically intended to encompass carrier materials approved for use in topical cosmetics. Representative carriers include water, oils, both vegetable and mineral, cream bases, lotion bases, ointment bases and the like. These bases include suspending agents, thickeners, penetration enhancers, and the like. Their formulation is well known to those in the art of cosmetics and topical pharmaceuticals. Additional information concerning carriers can be found in Part 8 of Remington's Pharmaceutical Sciences, 17th edition, 1985, Mack Publishing Company, Easton, Pennsylvania, which is incorporated herein by reference.

"Therapeutically effective dose" means a dose of a composition of this invention which, when applied topically to the skin of a patient afflicted with a dermatologic or other cosmetic or medical condition, or when administered by another route, results in an observable improvement in the patient's condition.

"Topical", when used to define a mode of administration, means that a material is administered by being applied to the skin.

"Topically effective" means that a material, when applied to the skin, produces a desired pharmacological result either locally at the place of application or systemically as a result of transdermal passage of an active ingredient in the material.

### The Oxy Group-bearing Aromatic Aldehydes

The oxy group-bearing aromatic aldehydes used in the present invention are selected from the group consisting of 2-ethoxybenzaldehyde, 4-ethoxybenzaldehyde, 4-propoxybenzaldehyde, 4-butoxybenzaldehyde, 4-pentyloxybenzaldehyde, and 4-hexyloxybenzaldehyde.

The aromatic aldehydes are generally employed as isolated compounds mixed with a suitable carrier.

### General Synthetic Procedures

The aromatic aldehydes employed in the compositions and methods of this invention are either known compounds or are compounds that can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

For example, such compounds are readily prepared by formulation of the corresponding aryl compound using, for example, a disubstituted formamide, such as *N*-methyl-*N-*phenylformamide, and phosphorous oxychloride (the Vilsmeier-Haack reaction), or using Zn(CN)₂ followed by water (the Gatterman reaction). Numerous other methods are known in the art for preparing such aryl carbonyl compounds. Such methods are described, for example, in I. T. Harrison and S. Harrison, *Compendium of Organic Synthetic Methods,* Wiley, New York, 1971, and references cited therein.

Certain aromatic aldehyde compounds can also be prepared by alkylation of the corresponding aryl hydroxy compound (e.g., 4-hydroxybenzaldehyde and the like). This reaction is typically conducted by contacting the aryl hydroxy compound with a suitable base, such as an alkali or alkaline earth metal hydroxide, fluoride or carbonate, in a inert solvent, such as ethanol, DMF and the like, to deprotonate the hydroxyl group. This reaction is generally conducted at about 0°C to about 50°C for about 0.25 to 2 hours. The resulting intermediate is then reacted *in situ* with about 1.0 to about 2.0 equivalents of an alkyl halide, preferably an alkyl bromide or iodide, at a temperature of from about 25°C to about 100°C for about 0.25 to about 3 days.

Additionally, various aromatic aldehydes can be prepared by reduction of the corresponding aryl nitriles. This reaction is typically conducted by contacting the aryl nitrile with about 1.0 to 1.5 equivalents of a hydride reducing agent, such as LiAlH(OEt)₃, in an inert solvent such as diethyl ether, at a temperature ranging from about -78° to about 25°C for about 1 to 6 hours. Standard work-up conditions using aqueous acid then provides the corresponding aryl aldehyde.

### Pharmaceutical and Cosmetic Compositions and Their Use

The oxy group-bearing aromatic aldehydes are administered in the form of a pharmaceutical or cosmetic composition. Such compositions can be prepared in manners well known in the pharmaceutical and cosmetic arts and comprise at least one active compound.

Generally, the compositions of this invention are administered in a cosmetic amount or a therapeutically effective dose. The amount of the compound actually administered in therapeutic settings may typically be determined by a physician, in the light of the relevant circumstances, including the condition to be treated, the chosen route of administration, the actual compound administered, the age, weight, and response of the individual patient, the severity of the patient's symptoms, and the like. In cosmetic settings the amount to be applied is selected to achieve a desired cosmetic effect.

The cosmetic compositions of this invention are to be administered topically. The pharmaceutical compositions of this invention are to be administered topically, transdermally or systemically such as orally or by injection.

In such compositions, the aromatic aldehyde compound is usually a minor component (from about 0.001 to about 20% by weight or preferably from about 0.01 to about 10% by weight) with the remainder being various vehicles or carriers and processing aids helpful for forming the desired dosing form.

Topical cosmetic forms and topical pharmaceutical dosing forms can include lotions, shampoos, soaks, gels, creams, ointments and pastes. Lotions commonly 5 employ a water or alcohol base. Gels are semi-solid emulsions or suspensions. Creams generally contain a significant proportion of water in their base while ointments and creams are commonly more oily.

Liquid forms, such as lotions suitable for topical administration or suitable for cosmetic application, may include a suitable aqueous or nonaqueous vehicle with buffers, suspending and dispensing agents, thickeners, penetration enhancers, and the like. Solid forms such as creams or pastes or the like may include, for example, any of the following ingredients, water, oil, alcohol or grease as a substrate with surfactant, polymers such as polyethylene glycol, thickeners, solids and the like. Liquid or solid formulations may include enhanced delivery technologies such as liposomes, microsomes, microsponges and the like.

The above-described components for liquid, semisolid and solid topical compositions are merely representative. Other materials as well as processing techniques and the like are set forth in Part 8 of Remington's Pharmaceutical Sciences, 17th edition, 1985, Mack Publishing Company, Easton, Pennsylvania.

When pharmaceutical compositions are to be administered transdermally they typically are employed as liquid solutions or as gels. In these settings the concentration of active aldehyde ranges from about 0.1% to about 20%, and preferably from about 0.1% to about 5%, of the composition with the remainder being aqueous mixed or nonaqueous vehicle, such as alcohols and the like, suspending agents, gelling agents, surfactant, and the like. Examples of suitable such materials are described below.

The aldehyde-containing compositions of this invention can also be administered in sustained release transdermal forms or from transdermal sustained release drug delivery systems. A description of representative sustained release materials can be found in the incorporated materials in Remington's Pharmaceutical Sciences.

The compositions for systemic administration include compositions for oral administration, that is liquids and solids, and compositions for injection.

Compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical occupant. Typical unit dosage forms include profiled, premeasured ampules or syringes of the liquid compositions or pills, tablets, capsules or the like in the case of solid compositions. In such compositions, the aromatic aldehyde is usually a minor component (from about 0.01 to about 20% by weight or preferably from about 0.1 to about 15% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an occupant such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As before, the aromatic aldehyde in such compositions is typically a minor component, often being from about 0.005 to 5% by weight with the remainder being the injectable carrier and the like.

The above-described components for orally administrable or injectable compositions are merely representative. Other materials as well as processing techniques and the like are set forth in the part of Remington's Pharmaceutical Sciences noted above.

The following formulation examples illustrate representative cosmetic and pharmaceutical compositions of this invention. The present invention, however, is not limited to the following pharmaceutical compositions.

### Formulation 1 - Liquid

A compound of 2-ethoxybenzaldehyde, 4-ethoxybenzaldehyde, 4-propoxybenzaldehyde, 4-butoxybenzaldehyde, 4-pentyloxybenzaldehyde, or 4-hexyloxybenzaldehyde (125 mg), and xanthan gum (4 mg) are blended , passed through a No. 10 mesh U.S. sieve, and then mixed with a previously made solution of microcrystalline cellulose and sodium carboxymethyl cellulose (11:89, 50 mg) in a water/isopropanol (75:25) mixture. Sufficient water/isopropanol are then added to produce a total volume of 5 mL.

### Formulation 2 - Cream

A commercial mineral oil-water cold cream base is obtained. To 100 grams of this base, 0.75 grams of a compound of 2-ethoxybenzaldehyde, 4-ethoxybenzaldehyde, 4-propoxybenzaldehyde, 4-butoxybenzaldehyde, 4-pentyloxybenzaldehyde, or 4-hexyloxybenzaldehyde as a fine powder or liquid is added with continuous mixing and stirring to suspend the powder in the base and yield a cosmetic or pharmaceutical composition.

This composition includes the following: deionized water (57.6% by weight); niacinamide (2.0%); glycerin (4.0%); phenonip (1.0%); propylene glycol (5.0%); transcutol (3.2%); jojoba Oil (3.5%); isocetyl alcohol (2.0%); isocetyl stearate (3.5%); mineral oil (3.0%); 4-ethoxybenzaldevyde (1.0%); isostearyl palmitate (3.0%); PEG-7 glyceryl cocoate (2.0%); Glycereth-7 (2.0%); POLYSORBATE-20^{™} (0.2%); cetyl ricinoleate (1.0%); glyceryl stearate/PEG-100 stearate (4.0%); and SEPIGEL^{™} (2.0%).

### Formulation 3 - Tablets

A compound of 2-ethoxybenzaldehyde, 4-ethoxybenzaldehyde, 4-propoxybenzaldehyde, 4-butoxybenzaldehyde, 4-pentyloxybenzaldehyde, or 4-hexyloxybenzaldehyde is mixed with dry gelatin binder and starch diluent in a 0.1:1:1 weight ratio. A lubricating amount of magnesium stearate is added and the mixture is tabletted into 210 mg tablets containing 10 mg of active aromatic aldehyde.

### Formulation 4 - Injection

A compound of 2-ethoxybenzaldehyde, 4-ethoxybenzaldehyde, 4-propoxybenzaldehyde, 4-butoxybenzaldehyde, 4-pentyloxybenzaldehyde, or 4-hexyloxybenzaldehyde is dissolved in injectable aqueous saline medium at a concentration of 1 mg/ml.

### Utility and Dosing

The compositions of this invention can be used topically to treat dermatological conditions such as
actinic keratosis,
acne,
allergic contact dermatitis,
atopic eczema,
contact dermatitis,
eczema,
erythema,
hand eczema,
itch,
irritant contact dermatitis,
psoriasis,
seborrhoric eczema,
rosacea,
alopecia areata,
damage from radiation, including UV radiation, IR radiation and any other ionizing radiation,
and the like.

The compositions, both cosmetic and pharmaceutical, can also be used to treat and prevent sunburn and to treat and prevent other forms of UV-induced inflammation and damage, and damage from other forms of ionizing radiation.

In these applications the cosmetic and pharmaceutical compositions are administered topically to achieve a desired cosmetic effect or a topical therapeutic effect.

In these uses the dose levels or application levels can be expressed in terms of the amount of active aromatic aldehyde delivered to the skin. For example, 1 to about 5 doses or applications per day, each containing from about 0.001 g to about 1 gram of active aldehyde can be used.

Alternatively, dose levels can be expressed in terms of the volume of formulated composition administered. For example, 1 to about 5 doses or applications per day, each containing from about 1 to about 30 grams of composition containing from about 0.01% to about 10% by weight of active aldehyde and especially from 0.02% to about 8% by weight.

When used in a sun care product, such as sun care lotion, the concentration of aldehyde can be as set forth above and the product can be applied as needed based on the intensity and duration of sun exposure.

Additionally, since the aromatic aldehydes have been discovered to effectively inhibit the release of cytokines, such a IL-1α, such compounds are useful for treating diseases characterized by an overproduction or a dysregulated production of cytokines, particularly IL-1α. Elevated levels of IL-1 and other cytokines are associated with a wide variety of inflammatory conditions, including rheumatoid arthritis, septic shock, erythema nodosum leprosy, septicemia, adult respiratory distress syndrome (ARDS), inflammatory bowel disease (IBD), uveitis, damage from ionizing radiation and the like.

The relationships between these cytokines and related materials and the inflammatory processes are described in more detail below at "Biology and Testing".

In the case of transdermal administration to treat such inflammatory conditions, one can administer a quantity of composition to a surface area of skin suitable to achieve an active aldehyde concentration in the systemic bloodstream of from about 0.5 to about 1000 micromolar and especially from about 1 to about 500 micromolar.

Injection dose levels for treating inflammatory conditions range from about 0.01 mg/kg/hour to at least 1 mg/kg/hour, all for from about 1 to about 120 hours and especially 24 to 96 hours. A preloading bolus of from about 0.01 mg/kg to about 1 mg/kg or more may also be administered to achieve adequate steady state levels.

With oral dosing, one to five and especially two to four and typically three oral doses per day are representative regimens. Using these dosing patterns, each dose provides from about 0.01 to about 10 mg/kg of the aromatic aldehyde, with preferred doses each providing from about 0.01 to about 5 mg/kg.

The aromatic aldehydes can be administered as the sole active agent or they can be administered in combination with other agents.

### Biology and Testing

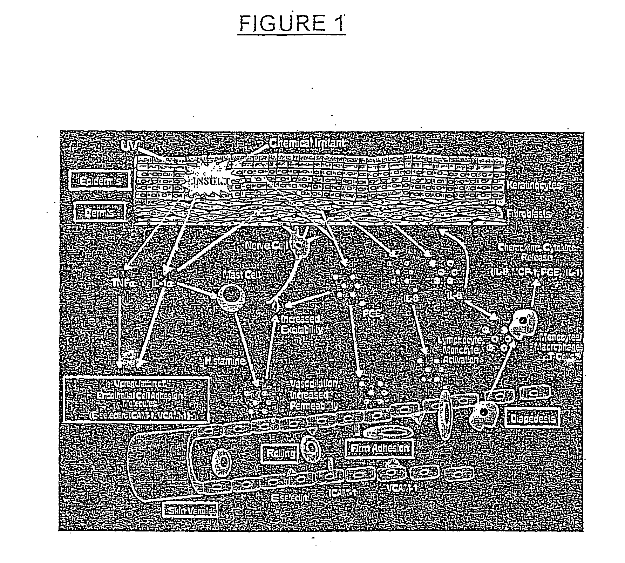

The examples include a number of *in vitro* studies to investigate the ability of these aldehydes to block various inflammatory processes in the skin. For these studies primary human keratinocytes and dermal fibroblast cell strains have been used as well as THP-1 monocytes and the Jurkat T-cell derived cell line. The *in vitro* experiments used to assess the anti-inflammatory activities of the aldehydes were selected on the basis of current knowledge about the skin inflammatory process. Fig. 1 depicts the events involved in cutaneous inflammation.

Inflammation in the skin is characterized by itching, pain, redness, swelling and, frequently, rough and flaky skin. These symptoms result from changes in blood flow to the site of inflammation, increased vascular permeability, the migration of cells from the circulation into the tissue, and the release of soluble mediators including cytokines, prostaglandins and chemokines. Skin inflammation can be triggered by: 1) infection caused by bacteria, parasites, fungi, or viruses, 2) injury resulting from physical trauma including burns, UV and ionizing radiation, 3) contact with chemical irritants, and 4) exposure to a foreign body such as an allergen which triggers an immune response.

Inflammation can be characterized as acute or chronic. Acute skin inflammation can result from exposure to UV radiation (UVR), ionizing radiation or contact with chemical irritants and allergens. In contrast, chronic inflammation results from a sustained immune cell mediated inflammatory response. Acute inflammatory responses are typically resolved within 1 to 2 weeks with little accompanying tissue destruction. Chronic inflammatory responses, however, are long-lasting because the antigen that triggered the response persists in the skin. This leads to continued recruitment of immune cells into the tissue, particularly T lymphocytes, which then produce and secrete high levels of many inflammatory mediators. Chronic inflammation leads to significant and serious tissue destruction.

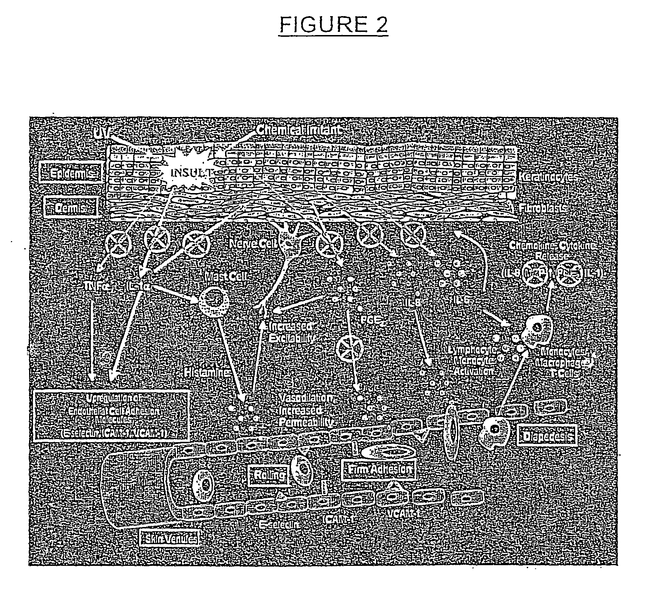

Regardless of the stimulus that triggers either an acute or chronic cutaneous inflammatory response, the initial events are similar and are shown in Figures 1 and 2. Triggering stimuli, such as UV radiation, induce keratinocytes in the skin to produce various cytokines including the key inflammatory cytokine, Interleukin-1 (IL-1). These cells also produce Tumor Necrosis Factor (TNF-α) and prostaglandin E2 (PGE-2). PGE-2 causes vasodilation of blood vessels near the site of injury and also increases the sensitivity of sensory nerve endings resulting in the sensation of itching and pain. The principal action of TNF-α is to increase the production of adhesion molecules on the surface of endothelial cells lining the blood vessels. These adhesion molecules act as anchors within the blood vessel allowing immune cells moving through the circulation to attach to the endothelium, an event that can lead to the diapedsis (movement) of these cells from the circulation and into the tissue. IL-1 produced by keratinocytes binds to specific receptors on fibroblasts within the dermis and activates signaling pathways that lead to the induction of many pro-inflammatory genes, such as those for COX-2, IL-8 and IL-6. IL-1 also binds to specific receptors on mast cells resulting in the production and secretion of histamine (which also increases nerve ending sensitivity), cytokines and other inflammatory mediators. In addition to responding to keratinocyte-derived IL-1, fibroblasts can also be directly activated by the triggering stimulus (e.g. UVR) and this further stimulates the expression of pro-inflammatory genes resulting in the production of PGE-2, the chemokine IL-8, as well as collagenase-1 (MMP-1). IL-8 stimulates diapedsis (chemotaxis, movement) of neutrophils, monocytes and ultimately lymphocytes from the endothelial cells where they have attached as a result of the TNF-α induced increase in adhesion molecules. Once in the tissue, neutrophils and monocytes produce additional cytokines (IL-1, IL-12), and chemokines including monocyte chemotactic protein (MCP-1), a potent chemokine that accelerates the movement of monocytes into the tissue and helps transform them into macrophages. Mature macrophages in turn produce a variety of matrix metalloproteinases (MMPs) that degrade extracellular matrix proteins and thus reduce the strength, elasticity and thickness of the skin.

If the inflammatory response is maintained by the continued presence of an antigen in the skin as is the case with chronic and destructive cutaneous diseases such as psoriasis and atopic dermatitis, the persistence of the antigen causes T-lymphocytes to enter the tissue site and become activated. This activation leads to the production of cytokines such as TNF-α, monocyte chemotactic protein-1 (MCP-1), IL-8, IL-12, and interferon-γ (INF-γ). Released IL-12 causes the T-lymphocytes to proliferate rapidly and to produce a wide range of cytokines, growth factors and other inflammatory mediators. These released products further activate macrophages, recruit monocytes, increase tissue destruction and cause accelerated and uncontrolled growth of skin cells, particularly keratinocytes. The result is pronounced skin inflammation with redness, pain, itching and scaling of the skin as the keratinocytes move rapidly to the surface and "flake off". Further, the rapid shedding of keratinocytes at the surface compromises the barrier function of the stratum corneum resulting in water loss and dry skin.

A common finding in inflammation is that cells in the skin respond to inflammatory stimuli by activating either one of two intracellular signaling pathways (or in some cases both pathways). These pathways are commonly referred to as the Stress Activated Kinase (SAK) pathway and the NF-kB pathway. The SAK pathway leads to the activation of the AP-1 transcription factor, which then binds to and activates several inflammatory genes including COX-2, IL-6 and MCP-1. Activation of the NF-kB pathway results in NF-kB protein translocation to the nucleus and activation of NF-kB driven inflammatory genes such as IL-8, MMP-1, TNF-α and the adhesion molecule, VCAM-1. Interestingly, many inflammatory genes including IL-1 have promoter elements that bind both AP-1 and NF-kB transcription factors and are thus regulated to some extent by both signaling pathways. The Cutanix screening assays are designed to determine which pathway is blocked by the compound under investigation, or if both pathways are effectively inhibited. A compound with the capacity to block the transcription of inflammatory genes regulated by each of these pathways will likely provide significant anti-inflammatory effects when applied topically. For each putative anti-inflammatory compound under consideration the initial screening program concentrates on the following target sites for intervention:
1. Inhibiting the production of IL-1 and PGE-2 in UVR or Tetradecanoyl Phorbol Acetate-treated keratinocytes.
2. Inhibiting the production of PGE-2 in UVR treated dermal fibroblasts.
3. Inhibiting the induction of PGE-2 in IL-1 treated fibroblasts.

Because one of the most common activators of skin inflammation is sunlight, specifically UVB radiation, the determination of a compound's ability to block the induction of pro-inflammatory PGE-2 by UVR in both keratinocytes and fibroblasts represents a logical first step in the screening process. In addition, because skin inflammation is often triggered by contact with chemical irritants or allergens, the use of TPA, which is known to trigger an inflammatory response in the skin, provides an additional model for the analysis of anti-inflammatory activities of test compounds. Finally, because IL-1 is one of the most important mediators and propagators of inflammation and is rapidly induced by an inflammatory stimulus, such as UVR, determining the ability of a potential anti-inflammatory compound to block either the production or action of IL-1 is a critically important initial screening study. As shown in Figs. 1 and 2, by blocking IL-1 production from keratinocytes, not only is the activation of fibroblasts suppressed but the activation of mast cells is also blocked thus preventing the release of histamine and other inflammatory mediators. Furthermore, inhibition of IL-1 production in the skin would prevent the activation of a large number of inflammatory genes that are stimulated solely by IL-1. These include COX-2, MMP-1, and a variety of cytokine and chemokine genes.

For all of the initial screening studies described herein, cells in culture are exposed to the appropriate agonist, (i.e. UVR, TPA or IL-1) and then incubated in medium for 24 or 48 hours in the presence or absence of the compound under investigation. At 24 and 48-hour time points, medium from the cells is removed and assayed for a number of inflammatory mediators by ELISA.

Only primary keratinocyte and fibroblast cell strains were used, not immortalized cell lines, for the screening studies. The use of normal cells from the skin increases the probability that results from *in vitro* studies will be predictive of effects of a given compound when applied topically.

Aldehydes that are found to completely (100%) suppress PGE-2 induction at a concentration of 100 micromolar or less are then subjected to more demanding dose-response studies including the following sequence of experiments:
1. Assessment by ELISA of a compound's ability to block a variety of UVR, TPA, or IL-1 induced inflammatory mediators in keratinocytes and fibroblasts including IL-6, TNF-α, IL-8, and MMP-1.
2. Assessment by ELISA of a compound's ability to block the production and secretion of inflammatory mediators by monocytes (THP-1 monocyte line) stimulated by lipopolysaccharide (LPS) and by T lymphocytes (Jurkat cells) stimulated with an antibody ligand that activates the cells.
3. The use of RPA (ribonuclease protection analysis) to determine if a compound is acting at the gene level to suppress the activity of specific inflammatory genes stimulated by exposure of cells to various agonists including UVR, IL-1, TPA, or LPS (lipopolysaccharide). Cutanix has developed a customized RPA "cocktail" for keratinocytes, fibroblasts, T-cells, and monocytes to simultaneously measure the expression of cell-type specific inflammatory genes in cells stimulated with UVR, IL-1, TPA or LPS in the presence or absence of the compound under investigation.
4. The use of microarray gene analysis to simultaneously examine the effect of any compound on the expression of more than 5,500 genes specific for cells present in the skin. The gene arrays used were purchased from Research Genetics and provide read-outs on genes known to be expressed in the skin.

The aldehydes can suppress a number of pro-inflammatory mediators and Fig. 2 identifies some of the events that are likely inhibited by the aldehydes in vivo (shown by the circled X).

### EXAMPLES

The following examples are provided to further describe the invention and are not intended as limitations on the scope of the invention which is defined by the appended claims.

### EXAMPLE 1

An initial *in vitro* experiment was conducted to demonstrate the activity of the aromatic aldehyde, 4-ethoxybenzaldehyde, ("4-EB") as a topically administered pharmaceutical.

For this experiment, human skin fibroblasts were seeded into 12 well culture dishes at a density of 80,000 cells/well in tissue culture medium and left overnight to attach to the dish. The next day, medium was removed and replaced with fresh medium containing either 1% ethanol as a diluent control, IL-1 at a concentration of 500 picograms/ml, or IL-1 plus 4-EB at either 250µM or 500µM. Cells were incubated for an additional 24 hours and at this time, the medium was removed and assayed by ELISA for the presence of PGE-2 in the culture medium. The results show that IL-1 caused a 17.8 fold increase in PGE-2 (control = 727 pg/10⁶ cells: IL-1 = 12,976 pg/10⁶ cells). However, cells treated with either concentration of 4-EB showed a complete inhibition of the IL-1 induction of PGE-2.

### EXAMPLE 2

*In vitro* experiments were conducted to demonstrate the activity of 3,5 di-*tert*-butyl, 4-hydroxybenzaldehyde, ("DTHB", not claimed) as a topically administered pharmaceutical

For this experiment, human skin fibroblasts were seeded into 12 well culture dishes at a density of 80,000 cells/well in tissue culture medium and left overnight to attach to the dish. The next day, medium was removed and replaced with fresh medium containing either 1% ethanol as a diluent control, IL-1 at a concentration of 500 picograms/ml, or IL-1 plus one of the compounds under investigation at a concentration of 1, 10, 50 or 100 µM. Cells were incubated for an additional 24 hours and at this time, the medium was removed and assayed by ELISA for the presence of PGE-2 in the culture medium. The results show that IL-1 caused a 4 to 22 fold increase in PGE-2.

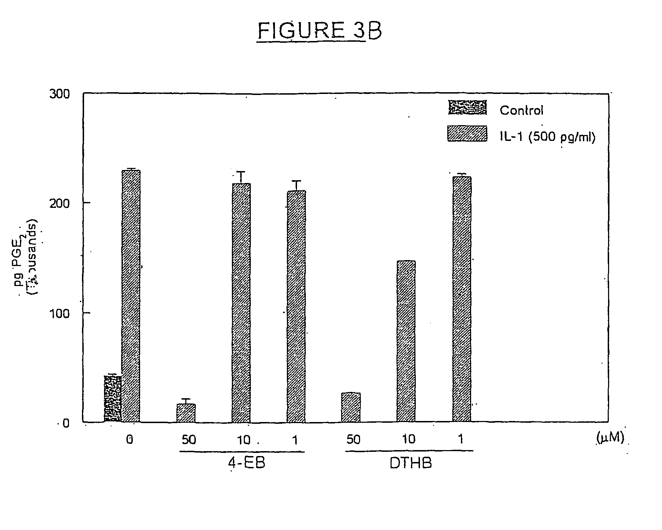

The detailed results of studies comparing the activity of DTHB to 4-ethoxybenzaldehyde ("4-EB"), are shown in Fig. 3B wherein the percent inhibitions are as follows: 4-EB, 100%, 6% and 10% at 50*µ*M, 10*µ*M and 1*µ*M; DTHB 100%, 44.4% and 3.3% at 50*µ*M, 10*µ*M and 1*µ*M.

### EXAMPLE 3

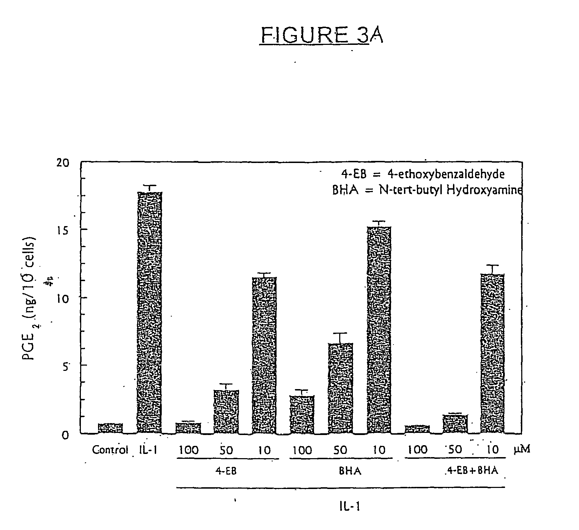

Subsequent studies were carried out to determine the dose-response of human skin fibroblasts to 4-EB. 4-EB completely blocked the IL-1 induction of PGE-2 at 100µM, blocked 82% of the PGE-2 induction at 50 µM, and blocked 35% at a concentration as low as 10µM. The results of the study are provided graphically in Fig. 3A.

### EXAMPLE 4

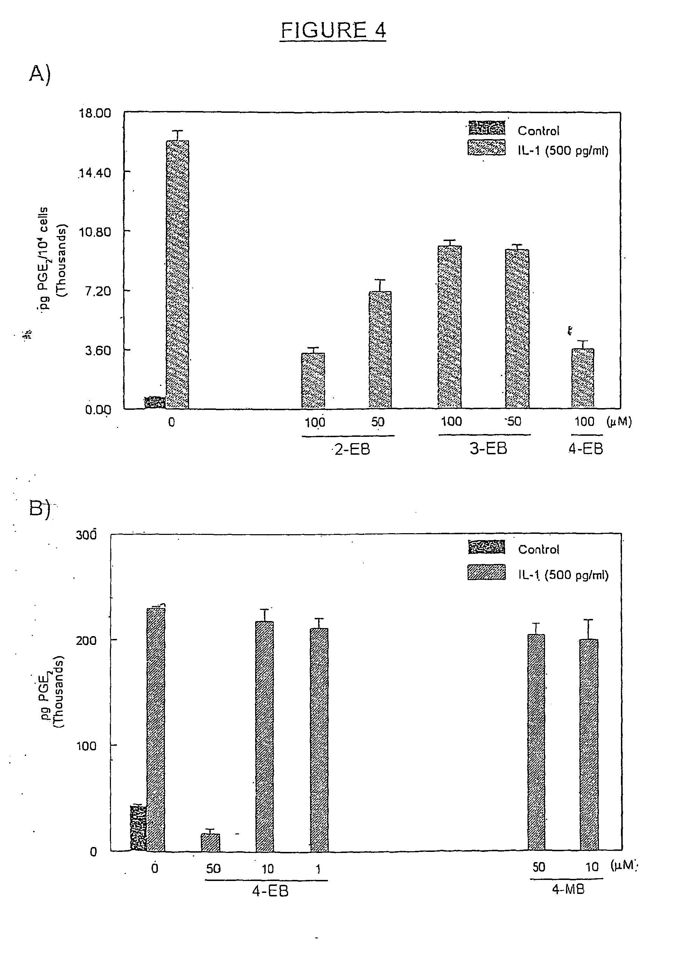

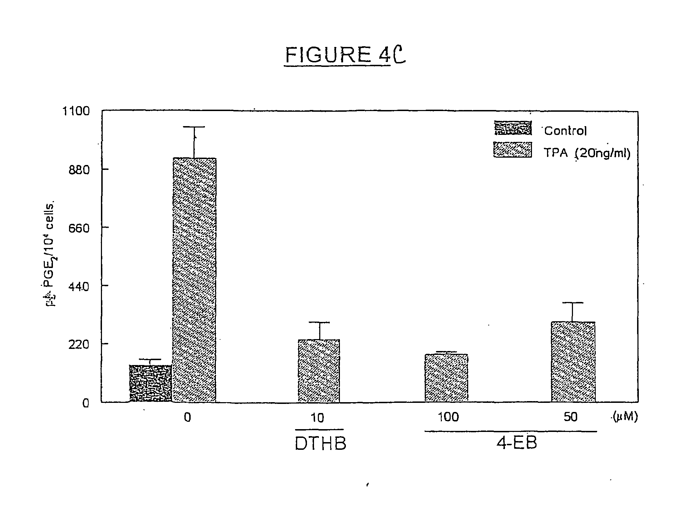

Similar *in vitro* studies as those described in Example 2 were run using human skin keratinocytes. The experimental set up was the same as described for Example 2, but replacing IL-1 with tetradecanoyl phorbol acetate (TPA) at a concentration of 32 nM as the agonist. Samples of 3,5-Di-*tert*-butyl, 4-hydroxybenzaldehyde (DTHB, not claimed) in concentrations of either 10, 50, or 100 µM were tested. The results show that TPA caused a 3.5 fold increase in PGE-2. However, treatment with DTHB blocked PGE-2 production by at least 50%. The detailed results of studies comparing DTHB to 4-EB are shown in Fig. 4C. The percent inhibitions are as follows: DTHB, 87.9% at 10µM; 4-EB, 94.9% and 79.9% at 100µM and 50µM.

### EXAMPLE 5

Subsequent *in vitro* experiments were conducted to demonstrate the activity of other aromatic aldehydes compared to the 4-ethoxybenzaldehyde, ("4-EB") as a topically administered pharmaceuticals. The compounds tested were 2-ethoxybenzaldehyde (2-EB), 3-ethoxybenzaldehyde (3-EB), and 4-methoxybenzaldehyde (4MB).

For this experiment, human skin fibroblasts were seeded into 12 well culture dishes at a density of 80,000 cells/well in tissue culture medium and left overnight to attach to the dish. The next day, medium was removed and replaced with fresh medium containing either 1% ethanol as a diluent control, IL-1 at a concentration of 500 picograms/ml, or IL-1 plus one of the compounds under investigation at a concentration of 1, 10, 50 or 100 *µ*M. Cells were incubated for an additional 24 hours and at-this time, the medium was removed and assayed by ELISA for the presence of PGE-2 in the culture medium. The results show that IL-1 caused a 4 to 22 fold increase in PGE-2.

Percent inhibitions as shown in the detailed results of Fig. 4A) are as follows: 2-EB, 82.9% and 58.9% at 100 *µ*M and 50 *µ*M; 3-EB, 41.2% and 42.6% at 100 *µ*M and 50 *µ*M; 4-EB, 81.5% at 100 *µ*M.

Concentrations of 10 or 50 *µ*M 4MB did not appear to inhibit the IL-1 induced production of PGE-2 in the fibroblasts. Percent inhibitions as shown in the detailed results of Fig. 4B) are as follows: 4-MB, 13.6% and 16.2% at 50µM and 10µM.

### EXAMPLE 6

Similar *in vitro* studies as those described in Example 5 were run using human skin keratinocytes. The experimental set up was the same as described for Example 5 but replacing IL-1 with tetradecanoyl phorbol acetate (TPA) at a concentration of 32 nM as the agonist. The compounds tested were 2-ethoxybenzaldehyde (2-EB), and 3-ethoxybenzaldehyde (3-EB) and 4-ethoxybenzaldehyde (4-EB) in concentrations of either 10, 50, or 100 µM. The results show that TPA caused a 3.5 fold increase in PGE-2. However, treatment with any of these compounds blocked PGE-2 production by at least 50%.

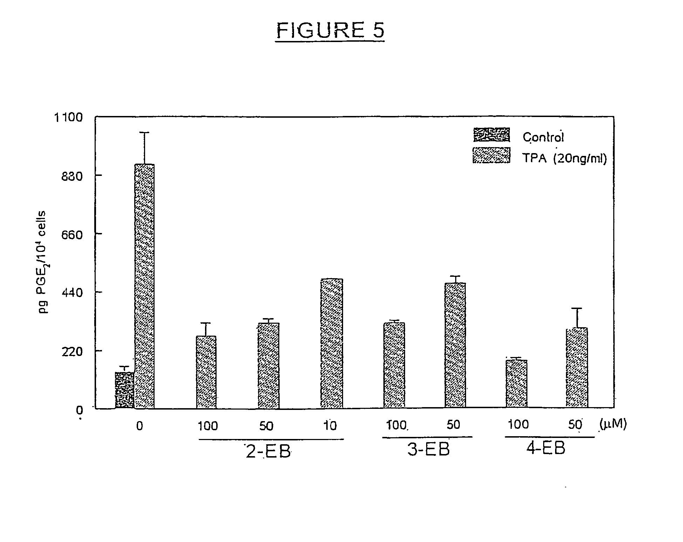

The percent inhibitions as shown in the detailed results in Fig. 5 are as follows: 2-EB, 83%, 76.6%, and 55.2% inhibition at 100 *µ*M, 50 *µ*M, and 10 µM; 3-EB, 76.7% and 57.7% at 100µM and 50µM; 4-EB, 94.9% and 79.9% at 100µM and 50µM.

### EXAMPLE 7

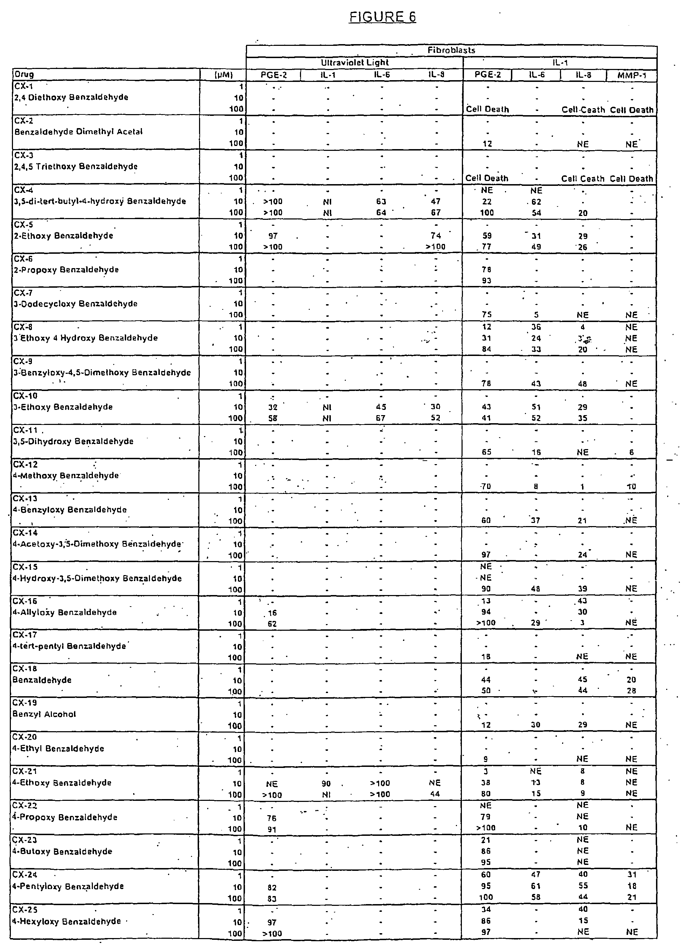

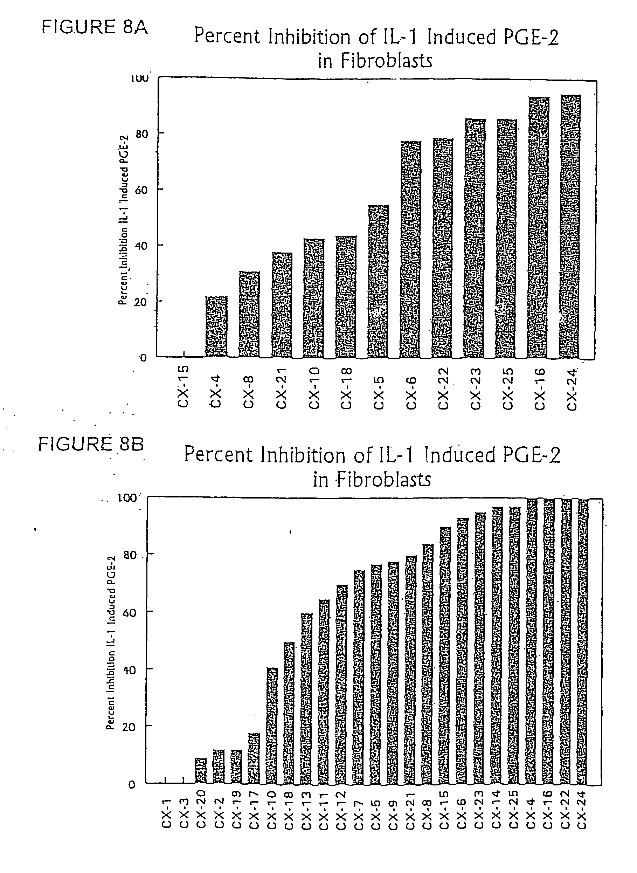

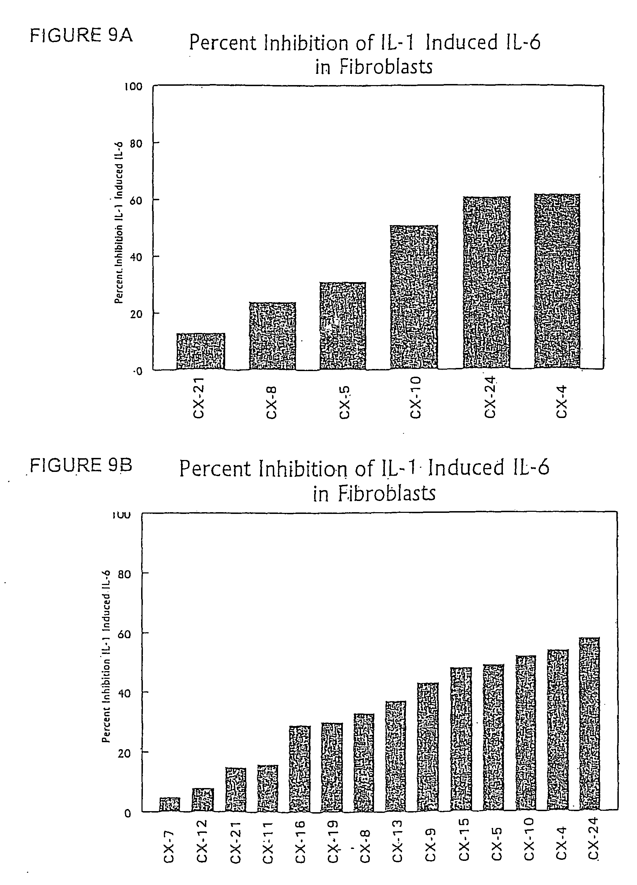

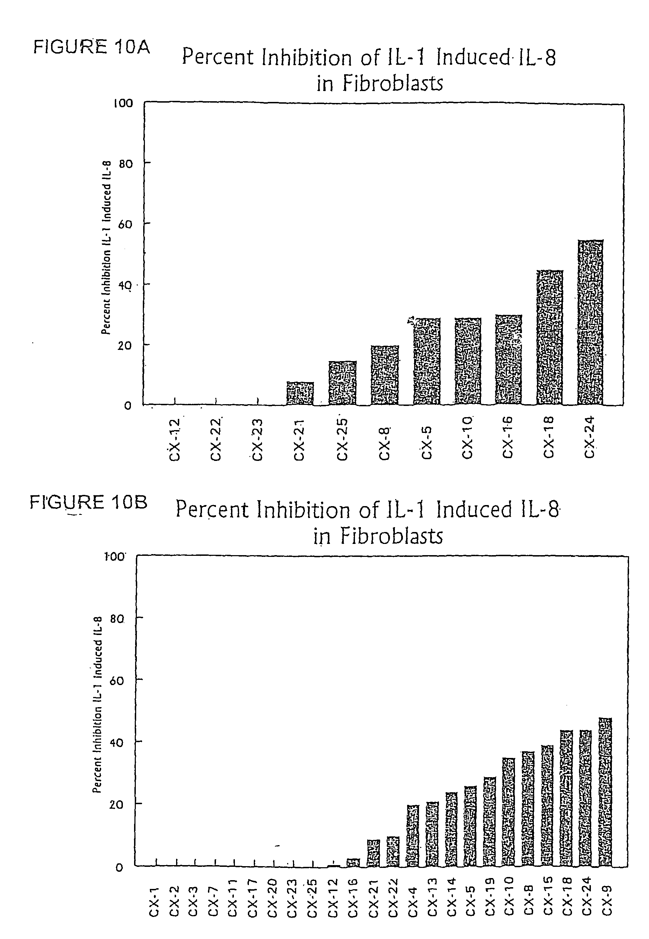

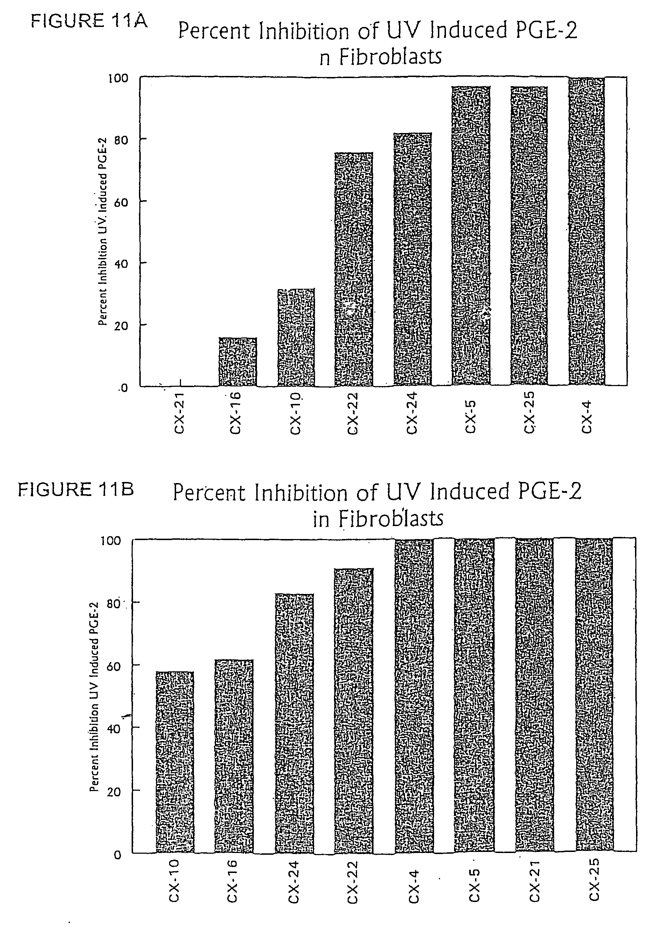

*In vitro* experiments were conducted to demonstrate the activity of a series of aromatic aldehydes as a topically administered pharmaceuticals. The compounds tested and the measured results are tabulated in Fig. 6, and shown graphically in Figs. 8-11. These data include results for aldehydes according to the invention and also include results for other related compounds.

For this experiment, human skin fibroblasts were seeded into 12 well culture dishes at a density of 80,000 cells/well in tissue culture medium and left overnight to attach to the dish. The medium was then replaced with PBS for a challenge with either UV-light or with IL-1. After irradiation or introduction of IL-1, the PBS was removed and culture medium containing the appropriate compound (or DMSO for controls) was then added and the cells cultured for an additional 24 hours. At that time, the medium was removed and assayed by ELISA for the presence of PGE-2, IL-1, IL-6, IL-8, or MMP-1 in the culture medium. The levels of protein in the conditioned medium were measured and reported as percent inhibition relative to diluent controls.

### IL-1 Challenge

On the second day, the medium was removed and replaced with fresh medium containing either 1% ethanol as a diluent control, EL-1 at a concentration of 500 picograms/ml, or IL-1 plus one of the compounds under investigation at a concentration of 100, 10, or 1 µM.

### UV-light Challenge

On the second day, the medium was removed and replaced with fresh PBS for irradiation. The fibroblasts were then irradiated with 50 mJ of UVB. UVB irradiation was obtained by illuminating the samples with an FS-20 sunlamp through the lids of the multi-well plates in order to filter out the UVC radiation. After irradiation the PBS solution was removed and replaced with a solution containing either 1% ethanol as a diluent control, or one of the aldehyde compounds at a concentration of 100, 10, or 1 µM. The cells were incubated for another 24 hours and the medium was then removed for the ELISA assays and the cells were counted.

### EXAMPLE 8

Similar *in vitro* studies as those described in Example 7 were run using human skin keratinocytes. The experimental set up was the same as described for Example 7. The products assayed by ELISA for the presence of PGE-2, IL-1, IL-6, IL-8, MMP-1, or TNF-α in the culture medium.

For the cells challenged by a biochemical agonist, IL-1 was replaced with tetradecanoyl phorbol acetate (TPA) at a concentration of 32 nM. When UV-light was used to challenge the cells, they were exposed to 75 mj of UVB, obtained by illuminating the samples with an FS-20 sunlamp through the lids of the multi-well plates in order to filter out the UVC radiation.

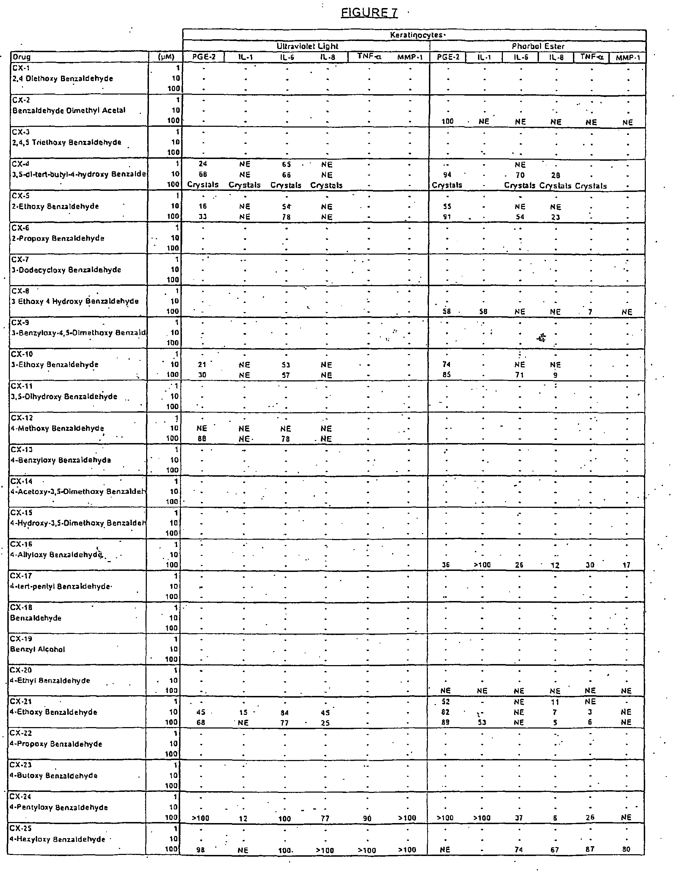

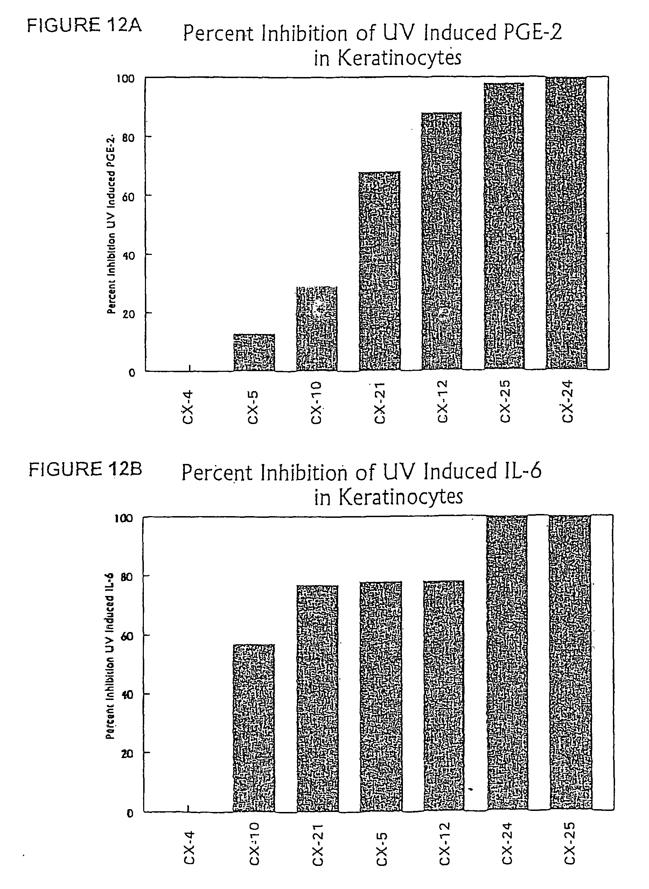

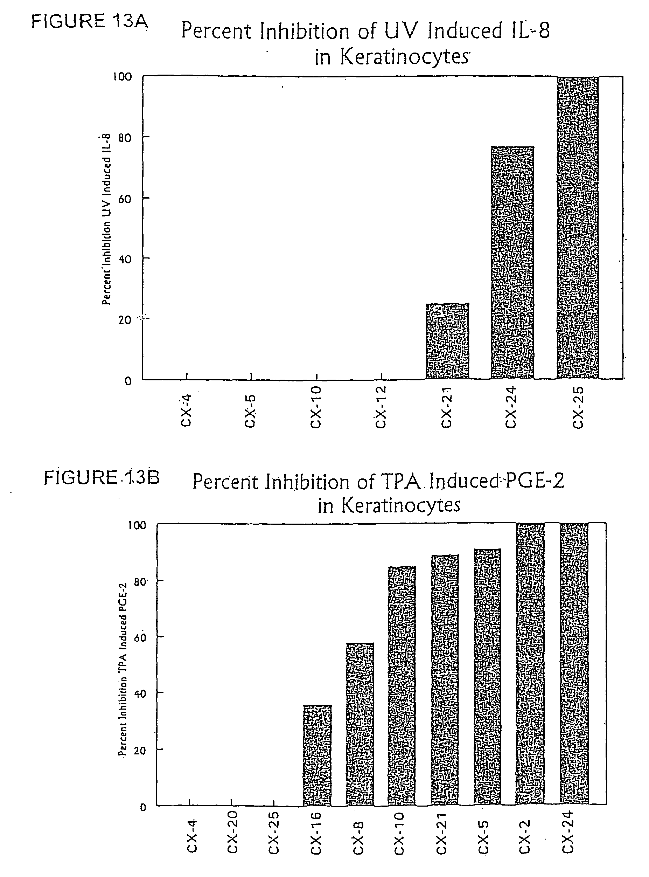

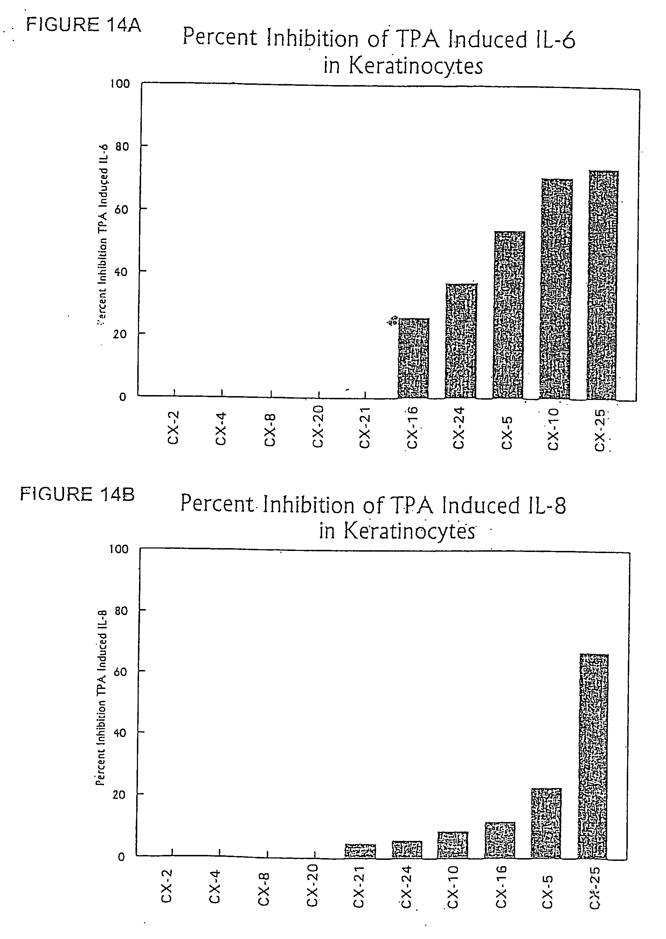

The compounds tested were in concentrations of either 100, 10, or 1 µM, and the protein expression levels are reported in percent inhibition relative to control treated cells. The measured percent inhibitions are tabulated in Fig. 7 and shown graphically in Figs. 12-14.

### EXAMPLE 9

Because of the marked anti-inflammatory effects seen when 4-EB was used in human fibroblast cell culture models, *in vivo* studies were carried out to determine if topically applied 4-EB could block an inflammatory response in humans. While the details provided herein are for a specific compound, the same tests can be used on any of the aromatic aldehydes of the present invention.

A topical lotion was developed for 4-EB which consists of the following:

| ***Aqueous phase*** | |
|---|---|
| Deionized water | 57.6% (by weight) |
| Niacinamide | 2.0% |
| Glycerin | 4.0% |
| Phenonip | 1.0% |

| ***Oil phase*** | |
|---|---|
| Propylene glycol | 5.0% |
| Transcutol | 3.2% |
| Jojoba Oil | 3.5% |
| Isocetyl alcohol | 2.0% |
| Isocetyl Stearate | 3.5% |
| Mineral Oil | 3.0% |
| 4-ethoxybenzaldehyde | 1.0% |
| Isostearyl Palmitate | 3.0% |
| PEG-7 Glyceryl Cocoate | 2.0% |
| Glycereth-7 | 2.0% |
| POLYSORBATE-20^{™} | 0.2% |
| Cetyl Ricinoleate | 1.0% |
| Glyceryl Stearate/ PEG-100 Stearate | 4.0% |

| ***Thickener*** | |
|---|---|
| SEPIGEL™ | 2.0% |

This lotion was then tested by Franz cell percutaneous absorption analysis to determine how much 4-EB could penetrate human skin over a 24 hour period. The lotion formulation above provided a flux rate of 4-EB through human skin of 30-50 micrograms/ hour.

This lotion was then tested to determine if it could prevent an inflammatory response when applied topically to human skin. For this study a lab volunteer was irradiated on a quarter sized spot on the inner forearm with 60-80 mJ of UVB light (a sunlamp). This dose was sufficient to cause a highly visible red erythema response. Immediately following irradiation on both arms, one arm was treated with the above 4-EB lotion while the other arm was treated with the same lotion formulation but with no 4-EB. Within 2-6 hours after irradiation the vehicle-treated arm developed a pronounced red erythema response at the site of irradiation while the 4-EB lotion treated spot did not. Even the next day, 14 hours post-irradiation, the spot treated with 4-EB showed no redness. This study demonstrates that topically applied 4-EB has marked anti-inflammatory activity.

In addition to its anti-inflammatory activity, compounds of the present invention, either alone or in combination with other compounds, such as ethyl vanillin, may have anti-aging properties. One of the classical symptoms of skin aging is an increase in collagenase activity in dermal fibroblasts which destroys collagen thereby leading to sagging skin and wrinkles.

### Implications of the Results in terms of Potential Uses of the Discovery

### Anti-aging

The finding that aromatic aldehydes of the present invention inhibit the activity of inflammatory genes in cultured skin cells and that they can block an inflammatory response *in vivo* when applied topically suggests wide utility for these compounds in the cosmetic, dermatology and oral drug markets. In the cosmetic market, these compounds when formulated for topical use can be expected to lower chronic sun-induced inflammation, which causes the activation of genes in skin cells that destroy the skin matrix. By inhibiting sun-induced genes such as MMP-1 (collagenase), gelatinase, and cytokines IL-1, IL-12, etc. 2-EB, and 4-EB will prevent the further breakdown of the skin and thus lessen the production of lines and wrinkles, sagging skin, and thinning of skin. It is likely that these aromatic aldehydes will stimulate genes that support the skin matrix such as collagen (studies ongoing). Thus, this product can be used as a "skin restorative" product for sun-damaged skin. It has its utility in treating actinic keratoses by both preventing their formation and actually reducing the size and number of existing keratoses.

### Sun Care Products

The finding that topically applied 4-EB, or any other compound of this invention, can completely prevent the onset of a sunburn by UVB exposure suggests the use of aromatic aldehydes in sun care products including pre-sun, sun-tan lotions, and after-sun products. It is not suggested that the molecules have sun-screen properties (which they probably do to some extent) but that they can actually arrest the progression of a sunburn AFTER the skin has already been exposed to the UV rays of the sun. Although it has been shown that topical application of the product inunediately after UVB exposure will prevent the onset of sunburn, it is also possible that application of the product even after the sunburn has appeared may: 1) prevent the continued progression of sunburn, and 2) reverse the redness already present.

### EXAMPLE 10

### Rosacea Clinical Study

The 30 subjects with mild to moderate rosacea were treated either with lotion containing 1% w 4-EB (20 subjects) or with a control lotion with the active material removed. The study was randomized and double blinded. During their first visit, patients were evaluated using 4 measurements of disease: 1) erythema, 2) desquamation (peeling), 3) uneven skin tone, and 4) dermatitis. The clinician also provided an "Overall Severity" score which ranged from 1-6 with 6 being the most severe level of overall disease. Patients were photographed to record the severity of the disease. After evaluation patients were sent home with either the test lotion or the control lotion and told to apply it morning and evening for two weeks. They then returned to the clinic for a two-week evaluation and at that time received more product for an additional 2 weeks. At four weeks, both the clinician and the subjects evaluated the severity of their disease. Digital photographs of the treated areas were also taken.

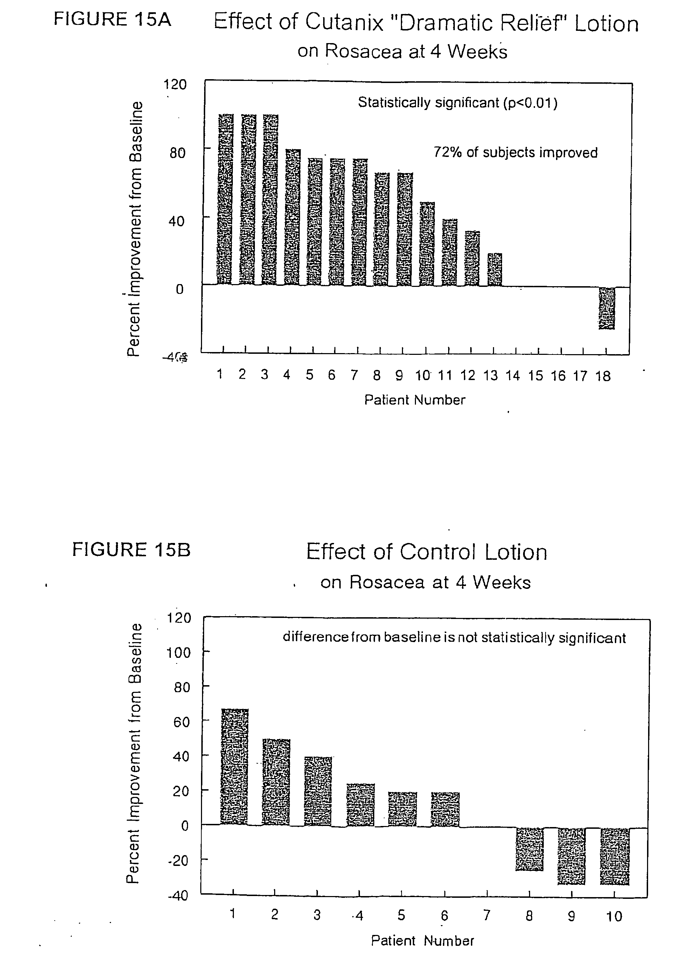

Of the 30 rosaceae patients that started the study, 28 completed the four-week period. None of the subjects, including those who dropped out, experienced any irritation or other adverse effect from the product. The bar graph of Fig. 15A summarizes the percentage improvement in "Overall Severity" for the test lotion treated group at 4 weeks. As can be seen, the severity of rosacea decreased in 13/18 subjects (72%). Average improvement among those responding was 68% (49% for all patients). This is a statistically significant result.

The bar graph of Fig. 15B summarizes the percentage improvement in "Overall Severity" for the control lotion treated group at 4 weeks. As can be seen, the severity of rosacea decreased in 6/10 subjects (60%) but increased in 3/10 (30%). Average overall improvement was 15% which is not a significantly significant result.

The test lotion also achieved another important statistical threshold in the rosacea study. The degree of improvement in the test lotion treated group was significantly better than the degree of improvement in the control treated group (p=0.05) using both Wilcoxon and Analysis of Variance statistics. These results are of sufficient quality to meet regulatory standards for drug efficacy and clearly establish the ability of 4-ethoxybenzaldehyde to suppress skin inflammation in humans.

Rosacea is a difficult disease to treat because of the severity of skin inflammation and vasodilation. Considering that a 2% formulation of 4-EB has been shown to be more effective in blocking UV-induced erythema than the 1% formulation used in this clinical study, a higher strength version of the test lotion may provide even greater efficacy in treating rosacea.

## Claims

1. A composition comprising a pharmaceutically or cosmetically acceptable topical carrier and a pharmaceutically or cosmetically effective amount of an aromatic aldehyde selected from the group consisting of 2-ethoxybenzaldehyde, 4-ethoxybenzaldehyde, 4-propoxybenzaldehyde, 4-butoxybenzaldehyde, 4-pentyloxybenzaldehyde, and 4-hexyloxybenzaldehyde.

2. The composition of claim 1 wherein the aromatic aldehyde is 2-ethoxybenzaldehyde.

3. The composition of claims 1 wherein the aromatic aldehyde is 4-ethoxybenzaldehyde.

4. The composition of claim 1 wherein the aromatic aldehyde is 4-propoxybenzaldehyde.

5. The composition of claim 1 wherein the aromatic aldehyde is 4-butoxybenzaldehyde.

6. The composition of claim 1 wherein the aromatic aldehyde is 4-pentyloxybenzaldehyde.

7. The composition of claim 1 wherein the aromatic aldehyde is 4-hexyloxybenzaldehyde.

8. The composition of any one of claims 1 to 7 wherein the composition is a cosmetic composition and wherein the compound is present in a cosmetically effective amount.

9. The cosmetic composition of claim 8 wherein the carrier is a liquid carrier.

10. The cosmetic composition of claim 8 wherein the carrier is a cream carrier.

11. The composition of any one of claims 1 to 7 wherein the composition is a topical pharmaceutical composition and wherein the compound is present in a pharmaceutically effective amount.

12. The pharmaceutical composition of claim 11 wherein the carrier is a liquid or a cream carrier.

13. The transdermal pharmaceutical composition of claim 12 wherein the pharmaceutical composition is a transdermal pharmaceutical composition and the compound is present in a transdermally effective amount.

14. The transdermal pharmaceutical composition of claim 13 wherein the carrier is a liquid carrier.

15. The transdermal pharmaceutical composition of claim 12 wherein the carrier is a cream carrier.

16. The transdermal pharmaceutical composition of claim 12 in a sustained release dosage form.

17. A systemic pharmaceutical composition comprising a systemically suitable carrier and a pharmaceutically effective amount of an aromatic aldehyde selected from the group consisting of 2-ethoxybenzaldehyde, 4-ethoxybenzaldehyde, 4-poropoxybenzaldehyde, 4-butoxybenzaldehyde, 4-pentyloxybenzaldehyde, and 4-hexyloxybenzaldehyde.

18. The composition of claim 17 wherein the aromatic aldehyde is 2-ethoxybenzaldehyde.

19. The composition of claim 17 wherein the aromatic aldehyde is 4-ethoxybenzaldehyde.

20. The composition of claim 17 wherein the aromatic aldehyde is 4-propoxybenzaldehyde.

21. The composition of claim 17 wherein the aromatic aldehyde is 4-butoxybenzaldehyde.

22. The composition of claim 17 wherein the aromatic aldehyde is 4-pentyloxybenzaldehyde.

23. The composition of claim 17 wherein the aromatic aldehyde is 4-hexyloxybenzaldehyde.

24. The composition of any one of claims 17 to 24 wherein the carrier is an injectable carrier.

25. The composition of any one of claims 17 to 24 wherein the carrier is an oral liquid carrier.

26. The composition of any one of claims 17 to 24 wherein the carrier is a solid.

27. The composition of claim 26 in a unit dosage form.

28. The composition of claim 27 wherein the unit dosage form is a capsule.

29. The composition of claim 27 wherein the unit dosage form is a pill.

## Patentansprüche

1. Zusammensetzung, umfassend einen pharmazeutisch oder kosmetisch annehmbaren topischen Träger und eine pharmazeutisch oder kosmetisch wirksame Menge eines aromatischen Aldehyds, der ausgewählt ist aus der Gruppe bestehend aus 2-Ethoxybenzaldehyd, 4-Ethoxybenzaldehyd, 4-Propoxybenzaldehyd, 4-Butoxybenzaldehyd, 4-Pentyloxybenzaldehyd und 4-Hexyloxybenzaldehyd.

2. Zusammensetzung nach Anspruch 1, in der der aromatische Aldehyd 2-Ethoxybenzaldehyd ist.

3. Zusammensetzung nach Anspruch 1, in der der aromatische Aldehyd 4-Ethoxybenzaldehyd ist.

4. Zusammensetzung nach Anspruch 1, in der der aromatische Aldehyd 4-Propoxybenzaldehyd ist.

5. Zusammensetzung nach Anspruch 1, in der der aromatische Aldehyd 4-Butoxybenzaldehyd ist.

6. Zusammensetzung nach Anspruch 1, in der der aromatische Aldehyd 4-Pentyloxybenzaldehyd ist.

7. Zusammensetzung nach Anspruch 1, in der der aromatische Aldehyd 4-Hexyloxybenzaldehyd ist.

8. Zusammensetzung nach irgendeinem der Ansprüche 1 bis 7, in der die Zusammensetzung eine kosmetische Zusammensetzung ist und in der die Verbindung in einer kosmetisch wirksamen Menge vorliegt.

9. Kosmetische Zusammensetzung nach Anspruch 8, in der der Träger ein flüssiger Träger ist.

10. Kosmetische Zusammensetzung nach Anspruch 8, in der der Träger ein Cremeträger ist.

11. Zusammensetzung nach irgendeinem der Ansprüche 1 bis 7, in der die Zusammensetzung eine topische pharmazeutische Zusammensetzung ist und in der die Verbindung in einer pharmazeutisch wirksamen Menge vorliegt.

12. Pharmazeutische Zusammensetzung nach Anspruch 11, in der der Träger ein flüssiger oder ein Cremeträger ist.

13. Transdermale pharmazeutische Zusammensetzung nach Anspruch 12, in der die pharmazeutische Zusammensetzung eine transdermale pharmazeutische Zusammensetzung ist und die Verbindung in einer transdermal wirksamen Menge vorliegt.

14. Transdermale pharmazeutische Zusammensetzung nach Anspruch 13, in der der Träger ein flüssiger Träger ist.

15. Transdermale pharmazeutische Zusammensetzung nach Anspruch 12, in der der Träger ein Cremeträger ist.

16. Transdermale pharmazeutische Zusammensetzung nach Anspruch 12 in einer Dosierungsform mit verzögerter Freisetzung.

17. Systemische pharmazeutische Zusammensetzung, umfassend einen systemisch geeigneten Träger und eine pharmazeutisch wirksame Menge eines aromatischen Aldehyds, der ausgewählt ist aus der Gruppe bestehend aus 2-Ethoxybenzaldehyd, 4-Ethoxybenzaldehyd, 4-Propoxybenzaldehyd, 4-Butoxybenzaldehyd, 4-Pentyloxybenzaldehyd und 4-Hexyloxybenzaldehyd.

18. Zusammensetzung nach Anspruch 17, in der der aromatische Aldehyd 2-Ethoxybenzaldehyd ist.

19. Zusammensetzung nach Anspruch 17, in der der aromatische Aldehyd 4-Ethoxybenzaldehyd ist.

20. Zusammensetzung nach Anspruch 17, in der der aromatische Aldehyd 4-Propoxybenzaldehyd ist.

21. Zusammensetzung nach Anspruch 17, in der der aromatische Aldehyd 4-Butoxybenzaldehyd ist.

22. Zusammensetzung nach Anspruch 17, in der der aromatische Aldehyd 4-Pentyloxybenzaldehyd ist.

23. Zusammensetzung nach Anspruch 17, in der der aromatische Aldehyd 4-Hexyloxybenzaldehyd ist.

24. Zusammensetzung nach irgendeinem der Ansprüche 17 bis 24, in der der Träger ein injizierbarer Träger ist.

25. Zusammensetzung nach irgendeinem der Ansprüche 17 bis 24, in der der Träger ein oraler flüssiger Träger ist.

26. Zusammensetzung nach irgendeinem der Ansprüche 17 bis 24, in der der Träger ein Feststoff ist.

27. Zusammensetzung nach Anspruch 26 in einer Einheitsdosierungsform.

28. Zusammensetzung nach Anspruch 27, in der die Einheitsdosierungsform eine Kapsel ist.

29. Zusammensetzung nach Anspruch 27, in der die Einheitsdosierungsform eine Pille ist.

## Revendications

1. Composition comprenant un support topique acceptable sur le plan pharmaceutique ou cosmétique et une quantité efficace sur le plan pharmaceutique ou cosmétique d'un aldéhyde aromatique choisi dans le groupe constitué par le 2-éthoxybenzaldéhyde, le 4-éthoxybenzaldéhyde, le 4-propoxybenzaldéhyde, le 4-butoxybenzaldéhyde, le 4-pentyloxybenzaldéhyde et le 4-hexyloxybenzaldéhyde.

2. Composition selon la revendication 1, dans laquelle l'aldéhyde aromatique est le 2-éthoxybenzaldéhyde.

3. Composition selon la revendication 1, dans laquelle l'aldéhyde aromatique est le 4-éthoxybenzaldéhyde.

4. Composition selon la revendication 1, dans laquelle l'aldéhyde aromatique est le 4-propoxybenzaldéhyde.

5. Composition selon la revendication 1, dans laquelle l'aldéhyde aromatique est le 4-butoxybenzaldéhyde.

6. Composition selon la revendication 1, dans laquelle l'aldéhyde aromatique est le 4-pentyloxybenzaldéhyde.

7. Composition selon la revendication 1, dans laquelle l'aldéhyde aromatique est le 4-hexyloxybenzaldéhyde.

8. Composition selon l'une quelconque des revendications 1 à 7, dans laquelle la composition est une composition cosmétique et dans laquelle le composé est présent dans une quantité efficace sur le plan cosmétique.

9. Composition cosmétique selon la revendication 8, dans laquelle le support est un support liquide.

10. Composition cosmétique selon la revendication 8, dans laquelle le support est un support crémeux.

11. Composition selon l'une quelconque des revendications 1 à 7, dans laquelle la composition est une composition pharmaceutique topique et dans laquelle le composé est présent dans une quantité efficace sur le plan pharmaceutique.

12. Composition pharmaceutique selon la revendication 11, dans laquelle le support est un support liquide ou crémeux.

13. Composition pharmaceutique transdermique selon la revendication 12, dans laquelle la composition pharmaceutique est une composition pharmaceutique transdermique et le composé est présent dans une quantité efficace sur le plan transdermique.

14. Composition pharmaceutique transdermique selon la revendication 13, dans laquelle le support est un support liquide.

15. Composition pharmaceutique transdermique selon la revendication 12, dans laquelle le support est un support crémeux.

16. Composition pharmaceutique transdermique selon la revendication 12 sous une forme posologique à libération prolongée.

17. Composition pharmaceutique systémique comprenant un support approprié sur le plan systémique et une quantité efficace sur le plan pharmaceutique d'un aldéhyde aromatique choisi dans le groupe constitué par le 2-éthoxybenzaldéhyde, le 4-éthoxybenzaldéhyde, le 4-propoxybenzaldéhyde, le 4-butoxybenzaldéhyde, le 4-pentyloxybenzaldéhyde et le 4-hexyloxybenzaldéhyde.

18. Composition selon la revendication 17, dans laquelle l'aldéhyde aromatique est le 2-éthoxybenzaldéhyde.

19. Composition selon la revendication 17, dans laquelle l'aldéhyde aromatique est le 4-éthoxybenzaldéhyde.

20. Composition selon la revendication 17, dans laquelle l'aldéhyde aromatique est le 4-propoxybenzaldéhyde.

21. Composition selon la revendication 17, dans laquelle l'aldéhyde aromatique est le 4-butoxybenzaldéhyde.

22. Composition selon la revendication 17, dans laquelle l'aldéhyde aromatique est le 4-pentyloxybenzaldéhyde.

23. Composition selon la revendication 17, dans laquelle l'aldéhyde aromatique est le 4-hexyloxybenzaldéhyde.

24. Composition selon l'une quelconque des revendications 17 à 24, dans laquelle le support est un support injectable.

25. Composition selon l'une quelconque des revendications 17 à 24, dans laquelle le support est un support liquide oral.

26. Composition selon l'une quelconque des revendications 17 à 24, dans laquelle le support est un solide.

27. Composition selon la revendication 26 sous une forme posologique unitaire.

28. Composition selon la revendication 27, dans laquelle la forme posologique unitaire est une capsule.

29. Composition selon la revendication 27, dans laquelle la forme posologique unitaire est une pilule.
